# EUROPEAN PATENT APPLICATION

(11) **EP 4 721 703 A1**
(43) Date of publication of application: **08.04.2026**
(21) Application number: 23939040.4
(22) Date of filing: 14.06.2023
(51) Int. Cl.: A61F 2/24

(54) **DEVICE, APPARATUS AND SYSTEM FOR HEART VALVE REPAIR**

(30) Priority: 31.05.2023 CN 202310634530
(71) Applicant: Kingstronbio (Changshu) Co., Ltd., Suzhou, Jiangsu 215000 (CN)
(72) Inventor: ZHONG, Shengping Sam, Suzhou, Jiangsu 215000 (CN); JIN, Yongfu, Suzhou, Jiangsu 215000 (CN)
(74) Representative: Chung, Hoi Kan
(86) International application number: PCT/CN2023/100198
(87) International publication number: WO 2024/244057

(57) **Abstract**

Disclosed in this disclosure are a device, an apparatus, and a system for repairing a heart valve. The device is used as a carrying tool for an annuloplasty ring when the annuloplasty ring is implanted into a valve annulus and includes a first carrier matched with the annuloplasty ring in terms of shape. A cord loop for connecting to artificial chordae tendineae is disposed on the first carrier. Two ends of the cord loop are in wound connection with one connection unit respectively, and a wound connection structure between the cord loop and the connection unit is fitted with a limiting structure disposed on the first carrier, so that the cord loop and the connection unit can be stably connected to the first carrier. When the connection unit is pulled out from the connection with the cord loop, the cord loop can be disconnected from the first carrier, so that the cord loop can be pulled out from the connection with the artificial chordae tendineae. In this disclosure, the artificial chordae tendineae can be separated from the cord loop without the need to cut the cord loop, so that the surgical difficulty of the implantation of the artificial chordae tendineae is reduced, the time required for surgical operations can be shortened, and possible safety risks resulting from cutting operations are avoided.

## Description

### TECHNICAL FIELD

This disclosure belongs to the technical field of heart valve repair, and specifically relates to a device, an apparatus, and a system for repairing chordae tendineae during heart valve repair surgery.

### BACKGROUND

In cases of heart valve disease, valvular regurgitation accounts for a certain proportion. The cause of valvular regurgitation often lies in the insufficiency of the valve leaflets, which causes blood to flow in reverse and consequently leads to insufficient blood supply. Apart from calcification of the valve leaflets, another major cause of insufficiency of the valve leaflets is dilation of the valve annulus, which leads to poor coaptation of the valve leaflets and rupture of the chordae tendineae and consequently causes prolapse of the valve leaflets.

For valvular regurgitation caused by poor coaptation of the valve leaflets due to dilation of the size of the valve annulus, the current common practice is to repair the heart valve by implanting an annuloplasty ring. The cause of valvular regurgitation caused by rupture of the chordae tendineae lies in the inability of the chordae tendineae to effectively restrain the valve leaflets after rupture, causing the valve leaflets to prolapse into the cardiac atrium during systole, which prevents the valve leaflets from coaptation and consequently causes regurgitation. In this case, the common practice is to implant artificial chordae tendineae to replace the ruptured chordae tendineae for repair. The implantation of artificial chordae tendineae is usually performed concurrently with the implantation of the annuloplasty ring. Generally, the implantation operation of artificial chordae tendineae follows the implantation of the annuloplasty ring. During the implantation of artificial chordae tendineae, the length of the chordae tendineae needs to be adjusted to adjust the coaptation state of the valve leaflets. In this process, the valve leaflets are usually first tensioned to the coaptation state. Then, by using the position of the ruptured chordae tendineae on the valve leaflets in this state as a reference, the artificial chordae tendineae connected to the papillary muscle are connected to the valve leaflets at this position, to ensure the accuracy of the implantation size of the artificial chordae tendineae between the valve leaflets and the papillary muscle. This accuracy has an important impact on the performance of the artificial chordae tendineae.

### SUMMARY

This disclosure aims to provide a device, an apparatus, and a system for repairing a heart valve, to assist in the implantation of artificial chordae tendineae during the implantation surgery of an annuloplasty ring of the heart valve, thereby ensuring the accuracy of the implantation size of the artificial chordae tendineae.

This disclosure is achieved through the following technical solutions:
A device for repairing a heart valve, where the device is used as a carrying tool for an annuloplasty ring when the annuloplasty ring is implanted into a valve annulus and includes a first carrier matched with the annuloplasty ring in terms of shape, and a cord loop for connecting to artificial chordae tendineae is disposed on the first carrier;
two ends of the cord loop are in wound connection with one connection unit respectively, and a wound connection structure between the cord loop and the connection unit is fitted with a limiting structure disposed on the first carrier, so that the cord loop and the connection unit can be stably connected to the first carrier; and
when the connection unit is pulled out from the connection with the cord loop, the cord loop can be disconnected from the first carrier, so that the cord loop can be pulled out from the connection with the artificial chordae tendineae.

In some embodiments, the limiting structure is a plurality of holes formed on the first carrier; one end of a flexible wire passes through the hole, after exiting from the hole, forms an intersection with the connection unit and winds around the connection unit, and then passes through the hole again and exits from the hole; and the flexible wire is connected to the connection unit at a position of the hole in a wire passing manner; and
the flexible wire is connected to the connection unit at positions corresponding to the holes along a wiring direction in the wire passing manner, and one cord loop with the two ends in wound connection with the connection unit respectively is formed between two adjacent holes respectively.

In some embodiments, a size of the structure formed by the wound connection between the cord loop and the connection unit is greater than a size of the hole, so that the wound connection structure between the cord loop and the connection unit can be clamped at a hole opening position at one end of the hole.

In some embodiments, one end of the hole is communicated to one side end surface on the first carrier for placing the annuloplasty ring, and the other end is communicated to the other opposite side end surface on the first carrier.

In some embodiments, the connection unit is disposed on one side end surface on the first carrier for placing the annuloplasty ring, and one end of the connection unit extends to the other opposite side end surface on the first carrier.

In some embodiments, the first carrier includes a horizontal portion and a vertical portion disposed along an inner side of the horizontal portion, a space for placing the annuloplasty ring is formed between the horizontal portion and the vertical portion, and the connection unit is disposed on one side on the first carrier for placing the annuloplasty ring and is located at a transition connection position between the horizontal portion and the vertical portion.

In some embodiments, the connection unit is a flexible member.

According to another aspect, this disclosure further provides an apparatus for repairing a heart valve, where the apparatus can be implanted into a valve annulus for repairing the valve annulus and includes a second carrier matched with the valve annulus in terms of shape, and a cord loop for connecting to artificial chordae tendineae is disposed on the second carrier;
two ends of the cord loop are in wound connection with one connection unit respectively, and a wound connection structure between the cord loop and the connection unit is fitted with a limiting structure disposed on the second carrier, so that the cord loop and the connection unit can be stably connected to the second carrier; and
when the connection unit is pulled out from the connection with the cord loop, the cord loop can be disconnected from the second carrier, so that the cord loop can be pulled out from the connection with the artificial chordae tendineae.

In some embodiments, the limiting structure is a plurality of holes formed on the second carrier; one end of a flexible wire passes through the hole, after exiting from the hole, forms an intersection with the connection unit and winds around the connection unit, and then passes through the hole again and exits from the hole; and the flexible wire is connected to the connection unit at a position of the hole in a wire passing manner; and
the flexible wire is connected to the connection unit at positions corresponding to the holes along a wiring direction in the wire passing manner, and one cord loop with the two ends in wound connection with the connection unit respectively is formed between two adjacent holes respectively.

According to another aspect, this disclosure further provides a system for repairing a heart valve, including a handle base and the device, where the handle base is connected to a first carrier of the device through a binding wire.

In some embodiments, connection structures are disposed at a plurality of positions on the first carrier along a circumferential direction thereof respectively, and one binding wire is adopted to be fitted with the connection structures along a wiring direction to fixedly connect the handle base to the first carrier.

Compared with the prior art, this disclosure has the following advantages and beneficial effects:
The cord loop is disposed on the first carrier or the second carrier, which can assist in the positioning of the implantation of the artificial chordae tendineae during the implantation surgery of the annuloplasty ring of the heart valve, thereby ensuring the accuracy of the implantation size of the artificial chordae tendineae.

The structure of disposing the cord loop on the second carrier is adopted, so that the possible impacts on the structure of the annuloplasty ring when the cord loop is disposed on the annuloplasty ring can be solved.

By improving the connection structure of the cord loop on the first carrier and the second carrier, the artificial chordae tendineae can be separated from the cord loop without the need to cut the cord loop, so that the surgical difficulty of the implantation of the artificial chordae tendineae is reduced, the time required for surgical operations can be shortened, and possible safety risks resulting from cutting operations are avoided.

### BRIEF DESCRIPTION OF THE DRAWINGS

To describe the technical solutions in the embodiments of this disclosure more clearly, the following briefly describes the accompanying drawings required for describing the embodiments. It should be understood that the following accompanying drawings show merely some embodiments of this disclosure, and therefore should not be regarded as limiting the scope. Those of ordinary skill in the art may still derive other related accompanying drawings from these accompanying drawings without creative efforts.
FIG. 1 is a schematic structural diagram of a use state of an implementation of a device for repairing a heart valve according to this disclosure;
FIG. 2 is a schematic structural diagram of a connection manner between a first carrier and a cord loop and a connection unit in a device for repairing a heart valve according to this disclosure;
FIG. 3 is a schematic structural diagram of a device for repairing a heart valve during implantation of artificial chordae tendineae according to this disclosure;
FIG. 4 is a schematic structural diagram of an implementation of a first carrier in a device for repairing a heart valve according to this disclosure; and
FIG. 5 is a schematic structural diagram of an implementation of a system for repairing a heart valve according to this disclosure.

In the drawings:
10. first carrier; 101. hole; 102. horizontal portion; 103. vertical portion; 104. connection hole;
20. cord loop;
30. connection unit;
40. handle base;
50. annuloplasty ring;
60. artificial chordae tendineae;
70. valve leaflet.

### DETAILED DESCRIPTION OF THE EMBODIMENTS

To make the objectives, technical solutions, and advantages of the embodiments of this disclosure clearer, the following will clearly and completely describe the technical solutions in the embodiments of this disclosure with reference to the accompanying drawings in the embodiments of this disclosure. Apparently, the described embodiments are some but not all of the embodiments of this disclosure.

In surgery for repairing a heart valve, an annuloplasty ring is implanted into a valve annulus to stabilize and reinforce the valve annulus. An implantation operation of the annuloplasty ring usually involves mounting the annuloplasty ring on a ring holder, and then using a handle to move the ring holder to an implantation position on the valve annulus. In implantation surgery of the annuloplasty ring, it is sometimes necessary to implant artificial chordae tendineae to replace ruptured chordae tendineae at the same time. The chordae tendineae are usually connected between papillary muscles and valve leaflets for tensioning the valve leaflets, thus limiting the valve leaflets. Therefore, when the artificial chordae tendineae are implanted, the implantation length of the artificial chordae tendineae between the papillary muscles and the valve leaflets directly affects the final effects that the artificial chordae tendineae can achieve, which has a significant impact on the performance of the artificial chordae tendineae. The implantation length of the artificial chordae tendineae needs to be determined in the implantation surgery.

In this disclosure, based on this requirement, by disposing a cord loop on the annuloplasty ring or the ring holder, in the implantation surgery of the artificial chordae tendineae, the artificial chordae tendineae are connected to the cord loop, the cord loop is used as a positioning reference to tension the valve leaflets, and the valve leaflets are tensioned to a coaptation state during normal working. In this case, the length of the artificial chordae tendineae is the length required for implantation, so that the implantation length of the artificial chordae tendineae can be accurately determined by using this method.

In an embodiment, the cord loop structure is disposed on an annuloplasty ring 50. The annuloplasty ring 50 is an apparatus for repairing a heart valve, and the apparatus is implanted into a valve annulus for repairing the valve annulus and includes a second carrier matched with the valve annulus in terms of shape. The second carrier is usually a D-shaped or C-shaped structural member that is closed or non-closed, and the second carrier may be an annuloplasty ring or artificial valve annulus of an existing structure. A plurality of cord loops 20 for connecting to artificial chordae tendineae are disposed on the second carrier, closed-loop structures are formed between the cord loops 20 and the second carrier, and the quantity of the cord loops on the second carrier is set according to actual requirements.

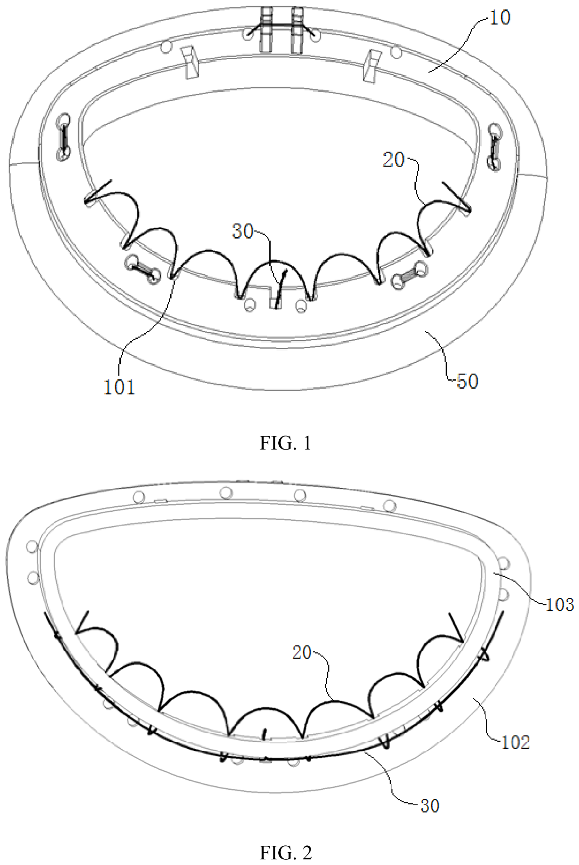

In terms of a connection manner adopted between the cord loops 20 and the second carrier, two ends of the cord loop are in wound connection with one connection unit respectively, and a wound connection structure of the cord loop and the connection unit is fitted with a limiting structure disposed on the second carrier, so that the cord loop and the connection unit can be stably connected to the second carrier. When the connection unit is pulled out from the connection with the cord loop, the cord loop can be disconnected from the annuloplasty ring, so that the cord loop can be pulled out from the connection with the artificial chordae tendineae. Specifically, a disposing structure of the cord loop on the second carrier can refer to a connection structure between a first carrier and the cord loop and the connection unit shown in FIG. 2. The first carrier in the figure can be regarded as the second carrier, and the second carrier is similar to the first carrier in FIG. 2 in terms of shape.

The characteristic of adopting this cord loop connection manner is that it is not necessary to cut off the cord loop when disconnecting the cord loop from the artificial chordae tendineae. This improvement is of great practical significance in the implantation surgery of the artificial chordae tendineae. It is known that heart valve repair surgery places high requirements on the convenience and safety of operating instruments due to limitations such as a small operating space and operation time. The performance of the operating instruments directly affects the operability of the surgery, the time required for the surgery, and the final effects that the surgery can achieve. It can be known that if the cord loop is disconnected from the artificial chordae tendineae by cutting off the cord loop, firstly, a cord loop cutting operation itself is difficult to perform in a confined operating space, secondly, the cutting operation will increase the time required for the surgery, and thirdly, since the cord loop generally is of a flexible structure, the cord loop will deform under stress, causing the position of a cutting point to change, which makes it easy to scratch the heart valve and consequently cause irreversible damage during cutting, or scratch the artificial chordae tendineae and consequently affect the use performance of the artificial chordae tendineae. Based on this problem encountered during surgical operations, the apparatus in this embodiment optimizes the connection manner of the cord loop on the second carrier. When the cord loop is disconnected from the artificial chordae tendineae, it is only necessary to pull out the connection unit from the connection with the cord loop. At this time, the cord loop can be disconnected from the second carrier, and then the cord loop is pulled out from the connection with the artificial chordae tendineae, thereby well solving the above problem.

In terms of the connection manner that can be adopted by the cord loop on the second carrier, a specific connection structure that can achieve the above function is given in the following embodiment. Certainly, a connection structure that can be adopted between the cord loop and the second carrier is not limited to this structural form.

Based on the purpose to be achieved by this disclosure, although the apparatus in the above embodiment solves the problems of providing a positioning reference for determining the length of the artificial chordae tendineae in the implantation surgery of the artificial chordae tendineae and of facilitating the disconnection of the cord loop from the artificial chordae tendineae, the apparatus, as the annuloplasty ring, needs to be implanted and remain in the human body to assist in the working of the valve annulus. Therefore, high requirements are put on the stability and reliability of the apparatus actually. In this case, if the cord loop is disposed on the annuloplasty ring, it is necessary to improve the structure thereof based on the existing annuloplasty ring to implement the connection and disposition of the cord loop. In this way, the complexity of the structure of the annuloplasty ring is increased, which not only affects the structural performance of the annuloplasty ring, but also increases a risk that may arise during the working of the annuloplasty ring in the human body.

Based on this consideration, in an embodiment, the cord loop 20 structure is disposed on a ring holder, which is a device for repairing a heart valve. The device is used as a carrying tool for an annuloplasty ring when the annuloplasty ring is implanted into a valve annulus, and the device includes a first carrier 10 matched with the annuloplasty ring in terms of shape. The first carrier 10 may be an existing ring holder, and a cord loop 20 for connecting to artificial chordae tendineae is disposed on the first carrier 10.

Two ends of the cord loop 20 are in wound connection with one connection unit 30 respectively, and a wound connection structure of the cord loop 20 and the connection unit 30 is fitted with a limiting structure disposed on the first carrier, so that the cord loop 20 and the connection unit 30 can be stably connected to the first carrier 10. When the connection unit is pulled out from the connection with the cord loop, the cord loop can be disconnected from the first carrier, so that the cord loop can be pulled out from the connection with the artificial chordae tendineae.

The characteristic of this connection structure between the first carrier and the cord loop and the connection unit is that when the connection unit is pulled out from the connection with the cord loop, the cord loop can be disconnected from the first carrier, so that the cord loop can be pulled out from the connection with the artificial chordae tendineae.

In other words, the improvements made to the cord loop structure herein mainly lie in the connection structure and connection manner between the cord loop structure and the first carrier. Taking one cord loop unit as an example, the two ends of the cord loop 20 are in wound connection with the connection unit 30 respectively. The wound connection herein can be understood as the cord loop being connected to the connection unit by winding around the connection unit. The characteristic of this connection manner lies in that a movable connection structure is formed between the cord loop and the connection unit. When the connection unit is pulled, the connection unit can be easily pulled out from the connection with the cord loop, that is, a specific quantity of wound turns can be arbitrary, as long as the above connection function can be achieved. Certainly, the wound connection herein can further be understood in a broader sense. For example, in addition to winding the cord loop around the connection unit, a portion, wound around the connection unit, of the cord loop forms a closed ring sleeved on the connection unit, which does not affect the achievement of the above connection function actually. Obviously, since the connection unit itself is movably connected to the first carrier, when the two ends of the cord loop are movably connected to the connection unit, the cord loop cannot be stably connected to the first carrier, and an integral closed-loop structure formed by the connection between the cord loop and the connection unit also cannot be stably connected to the first carrier. Therefore, the limiting structure is disposed on the first carrier 10 herein. While the cord loop is in wound connection with the connection unit, the cord loop is in fitted connection with the limiting structure at a connection position between the cord loop and the connection unit, so that the cord loop and the connection unit can be stably connected to the first carrier. Based on this connection structure, the characteristic of the connection structure further lies in that since the connection unit is movably connected to the first carrier and the cord loop at this time, when the connection unit is pulled out from the connection with the cord loop, the cord loop can be disconnected from the first carrier. At this time, the cord loop is separated from the first carrier, and the cord loop becomes an independent and free wire, so that the cord loop can be pulled out from the connection with the artificial chordae tendineae. In this way, during the surgery, the cord loop can be disconnected from the artificial chordae tendineae without the need to cut off the cord loop. An operating manner during the entire operation is just pulling out the connection unit and the cord loop from corresponding structures respectively, which obviously reduces the operating difficulty of the surgery.

In an embodiment, referring to FIG. 1, the limiting structure on the first carrier is a plurality of holes 101 formed on the first carrier 10. One end of a flexible wire passes through the hole 101, after exiting from the hole, forms an intersection with the connection unit 30 and winds around the connection unit 30, and then passes through the hole again and exits from the hole. The flexible wire is connected to the connection unit at the position of the hole in this wire passing manner. The flexible wire is connected to the connection unit 30 at positions corresponding to the holes along a wiring direction by adopting this wire passing manner, and one cord loop 20 with the two ends being in wound connection with the connection unit respectively is formed between two adjacent holes respectively. The adopted wire passing manner is as shown in FIG. 2. At this time, the cord loop and the connection unit can be stably connected to the first carrier.

Based on the principle implemented by the above structural function, to achieve the desired function using this connection manner, a functional portion of the formed cord loop and the connection unit generally need to be located at two ends of the hole respectively. Alternatively, the hole herein does not necessarily have to be a hole in the conventional sense and may also be a groove structure that allows the cord loop to pass through, and a portion that can separate the formed cord loop from the connection unit is disposed in the groove structure, thus preventing the entire structure composed of the cord loop and the connection unit from detaching from the first carrier. In other words, the limiting structure herein does not necessarily have to be a hole structure. A disposing position of the connection unit on the first carrier can also be set arbitrarily according to requirements, which is usually determined according to a specific disposing structure and position of the hole.

The connection unit 30 is generally a flexible member, for example, a flexible wire and rope, to facilitate pulling out the connection unit from the connection with the cord loop.

Usually, the cord loop needs to withstand a certain amount of force during the surgery. In this way, the cord loop can still be stably connected to the first carrier 10 under a certain amount of tensile force. Based on the connection structure and manner adopted between the cord loop 20 and the first carrier 10 in this embodiment, the two ends of the cord loop 20 are fitted with the connection unit and the hole, so that the two ends of the cord loop 20 can be fixed at the position of the hole through a friction force generated between each other. When subjected to a large tensile force, the cord loop will overcome the frictional force. At this time, there is a possibility that the two ends of the cord loop may slip, causing the cord loop to fail to provide a reliable connection for the artificial chordae tendineae. Therefore, the size of a structure formed by the wound connection between the cord loop and the connection unit is set to be greater than the size of the hole, so that the wound connection structure between the cord loop and the connection unit can be clamped at a hole opening position at one end of the hole, which can more effectively fix the cord loop. For example, in an example of setting the hole as a circular hole, when a wound connection manner shown in FIG. 2 is adopted between the cord loop and the connection unit, the size of the wound connection structure between the cord loop 20 and the connection unit 30 can be simplified to the sum of twice the diameter of the cord loop and the diameter of the connection unit. When the size is greater than the diameter of the hole, the cord loop can be stably fixed at a connection position. When the hole is set to a square hole, a size relationship on which the hole is based is the same.

The quantity of the holes herein is determined according to the quantity of the cord loops that need to be disposed. The disposing position of the hole on the first carrier 10 is also determined according to the disposing position of the cord loop on the first carrier.

In an embodiment, one end of the hole 101 is communicated to one side end surface on the first carrier 10 for placing the annuloplasty ring 50, and the other end of the hole is communicated to the other opposite side end surface on the first carrier. At this time, the connection unit 30 is disposed on the side end surface on the first carrier for placing the annuloplasty ring, and the side end surface limits the connection unit. Under this disposing state, one end of the connection unit 30 exits from a through hole formed on the annuloplasty ring and extends to the other opposite side end surface on the first carrier. The side end surface is usually oriented toward one side of the surgical operation. In this way, the connection unit can be conveniently pulled out from the first carrier 10 during the surgery, as shown in FIG. 1.

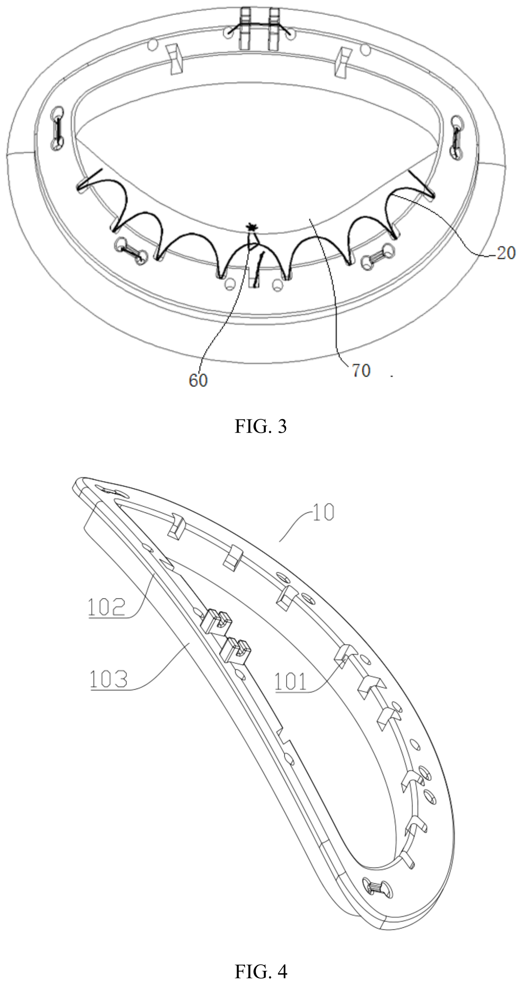

In an embodiment, the first carrier 10 includes a horizontal portion 102 and a vertical portion 103 disposed along an inner side of the horizontal portion. The horizontal portion 102 and the vertical portion 103 are disposed toward different directions respectively, so that a space for placing the annuloplasty ring is formed between the horizontal portion and the vertical portion, as shown in FIG. 4. The horizontal portion 102 forms an annular structure with an L-shaped cross section, and the horizontal portion 102 and the vertical portion 103 are used for limiting a part of the annuloplasty ring respectively. In the first carrier of this structure, the connection unit 30 is disposed on one side end surface on the first carrier for placing the annuloplasty ring, and the connection unit 30 is disposed at a transition connection position between the horizontal portion 103 and the vertical portion 103. In this way, this can be understood as the structure at the position on the first carrier being capable of limiting the connection unit more stably, and the resulting effect is obvious.

Usually, based on the function to be implemented by the cord loop, the cord loop 20 is disposed toward a central side of the ring holder. Based on this structural characteristic, the position of the hole on the first carrier 10 is set toward one side in a central direction of the first carrier, to facilitate the disposition of the cord loop on the first carrier. Meanwhile, based on the structural characteristic of the L-shaped cross section of the first carrier, the hole 101 on the first carrier 10 is actually similar to a groove structure formed at the transition connection position between the horizontal portion and the vertical portion, as shown in FIG. 4. The structural characteristics of the formed hole lie in that firstly, the cord loop can be better disposed toward the central direction of the first carrier, and secondly, when the first carrier is conveniently molded in an injection molding manner, the structure of a mold is simpler, and a hole structure can be molded on the first carrier at the same time, thereby reducing processing costs of the first carrier.

In another embodiment, the limiting structure is a plurality of holes 101 formed on the first carrier. Two ends of a flexible wire pass through two different holes 101 respectively, after exiting from the holes, form intersections with the connection unit and wind around the connection unit, and then pass through respective corresponding holes again and exit from the holes to form a cord loop with two ends in wound connection with the connection unit. This structural form is actually the same as that in the above embodiment in terms of the connection manner of the cord loop, and another possible manner of disposing the cord loop is just given herein. Under this condition, each cord loop may be one unit that is independent from each other.

In this implementation structure, the cord loops 20 can be connected at one end respectively, and diversified connection manners and connection structures can be adopted. The plurality of cord loops are connected into one wire having two free ends through the connection between the cord loops, to facilitate pulling out the cord loops from the artificial chordae tendineae. At this time, the connection between the cord loops may be arbitrary, as long as one complete wire can be formed. Certainly, a manner of sequentially connecting two adjacent cord loops at one end in order can be adopted generally.

Based on the connection structure and connection manner of the cord loop on the first carrier, in an embodiment, the cord loop is disposed on a second carrier by adopting the same structural form as described above. The adopted structure thereof can be that: A limiting structure on the second carrier is a plurality of holes formed on the second carrier. One end of a flexible wire passes through the hole, after exiting from the hole, forms an intersection with the connection unit and winds around the connection unit, and then passes through the hole again and exits from the hole. The flexible wire is connected to the connection unit at the position of the hole in this wire passing manner. The flexible wire is connected to the connection unit at positions corresponding to the holes along a wiring direction by adopting this wire passing manner, and one cord loop with the two ends being in wound connection with the connection unit respectively is formed between two adjacent holes respectively.

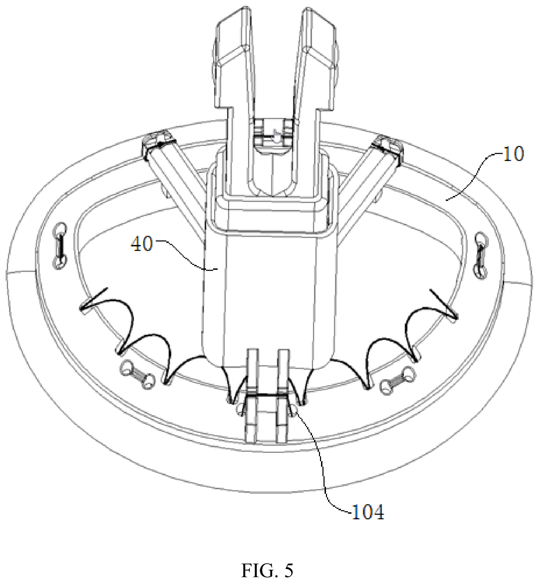

In another embodiment, as shown in FIG. 5, a system for repairing a heart valve includes a handle base 40. The handle base 40 is connected to a first carrier 10 of the device through a binding wire, to facilitate the separation between the handle base and the first carrier during surgery. In this way, a subsequent implantation operation of artificial chordae tendineae can be facilitated after an annuloplasty ring is implanted.

In terms of a specific connection manner adopted between the handle base and the first carrier, connection structures are disposed at a plurality of positions on the first carrier 10 along a circumferential direction thereof respectively. Taking structures shown in FIG. 4 and FIG. 5 as an example, the connection structures may be two connection holes 104 for the binding wire to pass through. During connection, by adopting one binding wire, along a wiring direction, and at the positions of the connection structures, the binding wire is fitted with the connection structures to fixedly connect three support legs of the handle base to the first carrier respectively. The advantage of this structure is that when the handle base is separated from the first carrier, the binding wire is cut only once, which undoubtedly plays a significant role in the entire surgical operation. In terms of connecting the three support legs of the handle base to the first carrier by adopting one binding wire, it is easily understood in combination with FIG. 5. For example, the binding wire passes through a connection hole of one connection structure, passes through another connection hole at the position after bypassing the support leg at the position, and then passes through one of the connection holes of the next connection structure. In this way, two free ends of the binding wire are fixed after passing through the connection holes sequentially. Certainly, a structure for limiting the wiring of the binding wire can be disposed on the first carrier, or a structure for limiting the binding wire can be disposed on the support leg, so that the binding wire can connect the handle base to the first carrier more stably.

Based on the structural characteristics of the above apparatus, during surgery, the artificial chordae tendineae 60 is connected to papillary muscles corresponding to original ruptured chordae tendineae first. A plurality of surgical sutures are adopted to pass through a valve annulus and then pass through the annuloplasty ring. The annuloplasty ring is pushed to the valve annulus. At this time, the artificial chordae tendineae pass through the middle of the annuloplasty ring. After an implantation position of the annuloplasty ring is adjusted, a scalpel is used to cut off a binding wire connecting the handle base to a ring holder, a handle and the handle base are taken out, and then the surgical sutures are knotted, to complete the implantation of the annuloplasty ring.

Referring to FIG. 3, since the handle base is taken away from the ring holder at this time, and a space on an inner side of the ring holder is completely exposed, a state of a valve leaflet can be clearly and completely observed. At this time, one end of the artificial chordae tendineae connected to the papillary muscles passes through a ruptured position of the original chordae tendineae on the valve leaflet and is connected to the valve leaflet 70, and then the artificial chordae tendineae are connected to a cord loop, the valve leaflet is tensioned through the cord loop, and the artificial chordae tendineae are knotted to be fixed on the valve leaflet. At this time, the implantation operation of the artificial chordae tendineae is completed. Then, a connection unit on the ring holder is pulled out, then the cord loop is pulled out from the ring holder, and then the cord loop is pulled out from a knot of the artificial chordae tendineae. Finally, a binding wire between the ring holder and the annuloplasty ring is cut off, and the ring holder is taken out, thereby completing the entire implantation operation.

In the description of this disclosure, it should be noted that the orientations or positional relationships indicated by the adopted terms "center", "up", "down", "left", "right", "vertical", "horizontal", "inside", "outside", and the like are in accordance with those shown in the accompanying drawings, or refer to orientations or positional relationships in which a product of this disclosure is typically placed when in use, and are intended only for the convenience of describing this disclosure and simplifying the description rather than for indicating or implying that the referred apparatus or element must be provided with a particular orientation or constructed and operated in a particular orientation; therefore, they should not be construed as limiting this disclosure.

In addition, the terms such as "horizontal" and "vertical" in the description of this disclosure do not imply that the component is required to be absolutely horizontal or suspended, but may be slightly tilted. For example, "horizontal" only refers to its direction being more horizontal relative to "vertical", and does not necessarily mean that the structure must be absolutely horizontal, but may be slightly tilted.

In the description of this disclosure, it should be further noted that, unless otherwise explicitly provided and limited, if there are terms such as "arranged", "mounted", "connected", and "connection", they should be understood in a broad sense, for example, it may be a fixed connection, a detachable connection, or an integral connection; it may be a mechanical connection or an electrical connection; it may be a direct connection or an indirect connection through an intermediate medium; and it may be a connection between two elements. For those of ordinary skill in the art, the specific meanings of the above terms in this disclosure may be understood based on specific circumstances.

The above descriptions are only preferred embodiments of this disclosure and are not intended to limit this disclosure in any way. Any simple modifications and equivalent changes to the above embodiments based on the technical essence of this disclosure shall fall within the protection scope of this disclosure.

## Claims

1. A device for repairing a heart valve, wherein the device is used as a carrying tool for an annuloplasty ring when the annuloplasty ring is implanted into a valve annulus and comprises a first carrier matched with the annuloplasty ring in terms of shape, and a cord loop for connecting to artificial chordae tendineae is disposed on the first carrier;
two ends of the cord loop are in wound connection with one connection unit respectively; and a wound connection structure between the cord loop and the connection unit is fitted with a limiting structure disposed on the first carrier, so that the cord loop and the connection unit can be stably connected to the first carrier; and
when the connection unit is pulled out from the connection with the cord loop, the cord loop can be disconnected from the first carrier, so that the cord loop can be pulled out from the connection with the artificial chordae tendineae.

2. The device for repairing a heart valve according to claim 1, wherein the limiting structure is a plurality of holes formed on the first carrier; one end of a flexible wire passes through one hole, after exiting from the hole, forms an intersection with the connection unit and winds around the connection unit, and then passes through the hole again and exits from the hole; and the flexible wire is connected to the connection unit at a position of the hole in the above wire passing manner; and
the flexible wire is connected to the connection unit at positions corresponding to the holes along a wiring direction in the wire passing manner; and one cord loop with the two ends in wound connection with the connection unit respectively is formed between two adjacent holes respectively.

3. The device for repairing a heart valve according to claim 1, wherein a size of the structure formed by the wound connection between the cord loop and the connection unit is greater than a size of the hole, so that the wound connection structure between the cord loop and the connection unit can be clamped at a hole opening position at one end of the hole.

4. The device for repairing a heart valve according to claim 1, wherein one end of the hole is communicated to one side end surface on the first carrier for placing the annuloplasty ring, and the other end is communicated to the other opposite side end surface on the first carrier.

5. The device for repairing a heart valve according to claim 4, wherein the connection unit is disposed on one side end surface on the first carrier for placing the annuloplasty ring, and one end of the connection unit extends to the other opposite side end surface on the first carrier.

6. The device for repairing a heart valve according to claim 1, wherein the first carrier comprises a horizontal portion and a vertical portion disposed along an inner side of the horizontal portion, a space for placing the annuloplasty ring is formed between the horizontal portion and the vertical portion, and the connection unit is disposed on one side on the first carrier for placing the annuloplasty ring and is located at a transition connection position between the horizontal portion and the vertical portion.

7. The device for repairing a heart valve according to any one of claims 1 to 6, wherein the connection unit is a flexible member.

8. An apparatus for repairing a heart valve, wherein the apparatus can be implanted into a valve annulus for repairing the valve annulus and comprises a second carrier matched with the valve annulus in terms of shape, and a cord loop for connecting to artificial chordae tendineae is disposed on the second carrier; and
two ends of the cord loop are in wound connection with one connection unit respectively; and a wound connection structure between the cord loop and the connection unit is fitted with a limiting structure disposed on the second carrier, so that the cord loop and the connection unit can be stably connected to the second carrier; and
when the connection unit is pulled out from the connection with the cord loop, the cord loop can be disconnected from the second carrier, so that the cord loop can be pulled out from the connection with the artificial chordae tendineae.

9. A system for repairing a heart valve, comprising a handle base and the device according to any one of claims 1 to 7, wherein the handle base is connected to a first carrier of the device through a binding wire.

10. The system for repairing a heart valve according to claim 9, wherein connection structures are disposed at a plurality of positions on the first carrier along a circumferential direction thereof respectively, and one binding wire is adopted to be fitted with the connection structures along a wiring direction to fixedly connect the handle base to the first carrier.
